# DEMANDE DE BREVET EUROPEEN

(11) **EP 0 813 846 A1**
(43) Date de publication de la demande: **29.12.1997**
(21) Numéro de dépôt: 97401434.2
(22) Date de dépôt: 20.06.1997
(51) Int. Cl.: A61B 17/86, A61B 17/68

(54) **Broche d'ostéosynthèse**

(30) Priorité: 20.06.1996 FR 9607911
(71) Demandeur: Khenifar, Brahim, 34600 Bedarieux (FR)
(72) Inventeur: Khenifar, Brahim, 34600 Bedarieux (FR)
(74) Mandataire: Wagret, Frédéric

(57) **Abrégé**

L'invention concerne une broche destinée à réunir et immobiliser des fragments osseux après fracture.

Selon l'invention, la broche est constituée d'une part d'une tige cylindrique (1) apte à être introduite dans l'os ou partie d'os concerné à partir de son extrémité de perforation profilée à cet effet, et d'autre part d'un capuchon de protection (2) apte à être engagé sur l'extrémité terminale de ladite tige opposée à son extrémité d'introduction, après mise en place de la tige; ladite tige comporte au moins sur ladite extrémité terminale des inégalités de relief sous forme de cannelures ou gorges circulaires (1E), transversales par rapport à l'axe de la tige et le capuchon (2), constitué d'un fond et de parois (2C) en forme de jupe, apte à s'emboîter sur ladite extrémité, est prévu avec des parois déformables diamétralement, ainsi propres à s'ouvrir pour s'engager sur ladite extrémité et à s'y fixer en se refermant par sertissage autour d'une gorge (1E); à cet effet, le capuchon comporte des fentes longitudinales sur ses parois.

Application à une broche d'ostéosynthèse utilisable en chirurgie traumatologique.

## Description

L'invention se rattache au secteur technique des moyens mécaniques pour réunir des fragments osseux d'une fracture quelconque.

Actuellement, les broches d'ostéosynthèse utilisées, quel que soit le type de fracture à traiter, sont difficilement protégées à leur extrémité opposée à celle d'engagement dans la partie considérée. En effet au moment de la mise en place de la ou des broches, le praticien ne connaît pas exactement la longueur nécessaire de broche, qui va dépendre de l'épaisseur de l'os, de sa longueur et autres éléments à prendre en considération. Il y a donc une longueur de broche introduite dans l'os, qui est variable.

Il convient de procéder au sectionnement de la partie débordante de la broche, généralement au moyen d'une pince coupante.

Cette partie coupée constitue une zone agressive qui peut être dangereuse pour les tendons dans le cas notamment d'une fracture du poignet; des risques apparaissent également pour les nerfs, les vaisseaux ou la peau, étant donné que des perforations cutanées ne sont pas à exclure.

On a proposé d'équiper l'extrémité débordante de la broche, d'un système de capuchon, fixé notamment au moyen d'une vis micrométrique latérale; mais la mise en place de cette vis est longue, fastidieuse et délicate et n'exclut pas le risque de départ du capuchon.

Le Brevet US 5 300 075 propose la mise en place d'un capuchon sur l'extrémité sectionnée de la broche, par rotation et engagement à force, les barbes latérales venues du sectionnement de la broche s'incrustant dans la paroi du capuchon; cette mise en place brutale est difficile à réaliser en présence de sang et de tissus sous-cutanés; et de plus elle est irréversible.

Le Brevet US 4 688 560 propose la mise en place d'un capuchon de protection constitué d'un fil enroulé en hélice conique ou d'un capuchon à ergots intérieurs; dans les deux cas la présence de parties déformées et débordantes sur l'extrémité sectionnée de la broche rend très délicate, sinon impossible l'engagement du capuchon, prévu pour une tige lisse.

L'invention vise à remédier aux difficultés rencontrées par la mise en place d'un capuchon de protection sur l'extrémité de la broche, rendue irrégulière et écrasée par le sectionnement et débordant latéralement en s'opposant ainsi à la mise en place du capuchon, calibré pour s'engager exactement sur la section de la tige. Un autre objet de l'invention est de prévoir un capuchon de protection qui puisse être solidarisé de façon fiable et sure, mais réversible. sur l'extrémité réceptrice de la broche, par un mouvement simple et rapide, compatible avec les conditions opératoires.

A cet effet l'invention concerne une broche d'ostéosynthèse du type constitué d'une part d'une tige cylindrique apte à être introduite dans l'os ou partie d'os concerné, à partir de son extrémité de perforation profilée à cet effet, et d'autre part d'un organe de protection constitué d'un capuchon apte à être engagé sur l'extrémité terminale de la dite tige, opposée à son extrémité d'introduction, après mise en place, et caractérisée a) en ce que la dite tige comporte au moins sur la dite extrémité terminale des inégalités de reliefs sous forme de cannelures ou gorges circulaires, régulièrement espacées et transversales par rapport à l'axe de la tige, et b) en ce que le capuchon, constitué d'un fond et de parois en forme de jupe apte à s'emboîter sur l'extrémité de la tige, est prévu avec des parois déformables diamétralement et ainsi propres à s'ouvrir pour s'engager et se fixer par pression sur ladite extrémité.

D'autres caractéristiques et avantages de l'invention ressortiront de la description qui suit et qui est donnée en relation avec diverses formes de réalisation de l'invention en référence aux dessins annexés.

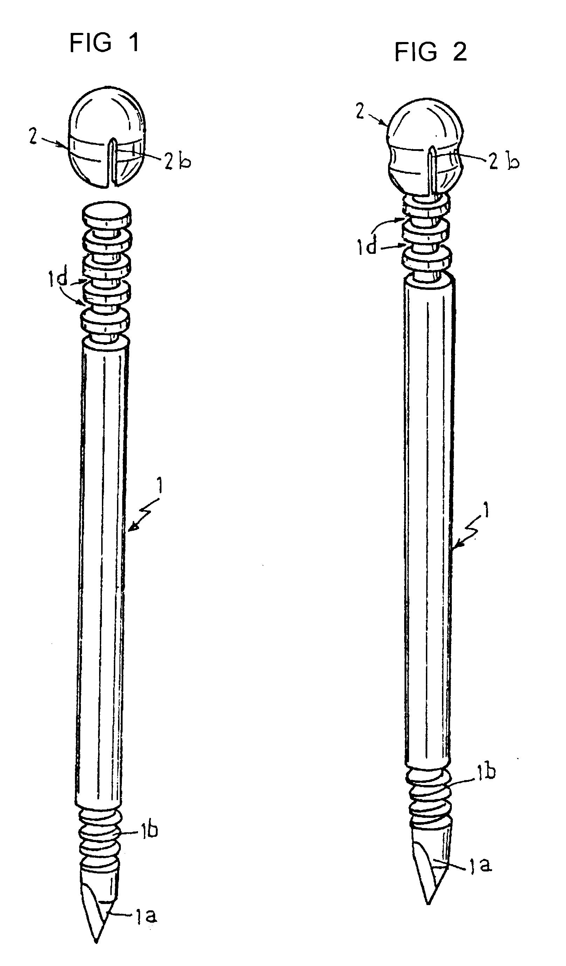

La figure 1 est une vue en perspective de la broche avant adaptation du capuchon de protection.
La figure 2 est une vue identique à la figure 1 après adaptation du capuchon protecteur;
La figure 3 est une vue partielle en coupe longitudinale montrant la mise en place du capuchon protecteur par effet de clip.
La figure 4 est une vue montrant la broche mise en place dans le cas d'une fracture du poignet.
La figure 5 est une vue en coupe d'une variante de réalisation de la broche selon l'invention, tandis que la figure 6 est une vue agrandie du capuchon de protection correspondant et vu en perspective.
La figure 7 est une vue en coupe de l'extrémité sectionnée de la broche avec le capuchon de protection en position pour son engagement, la figure 8 représentant les mêmes, le capuchon étant alors en cours d'engagement, et la figure 9 représente l'ensemble dans la position finale du bouchon, serti sur une gorge de la tige. Les figures 10 à 15 représentent des exemples d'utilisation du dispositif selon l'invention.

Selon l'ensemble des figures, la broche d'ostéosynthèse est exécutée , de façon connue en soi, à partir d'une tige cylindrique (1) dont l'une des extrémités (1a) est conformée pour être introduite dans la partie d'os considérée. Cette extrémité (1a) est par exemple convenablement taillée et profilée pour constituer une zone de perforation dans l'os. De même cette partie (1a) peut être suivie d'une portée filetée (1b) pour assurer, toujours de façon connue, l'ancrage de l'ensemble de la broche (1)

Selon l'invention au moins l'extrémité de la tige opposée à l'extrémité (1a) présente des cannelures ou gorges circulaires (1d, 1E), espacées régulièrement et transversalement par rapport à l'axe de la tige et sur une portée de cette dernière et ces gorges sont séparées par des collerettes (1e, 1F) de diamètre (d) correspondant au diamètre courant de la tige; une partie de cette tige, pourvue des cannelures ou gorges circulaires, peut être sectionnée pour adapter la longueur de la tige au cas à traiter; le sectionnement sera facilité étant opéré au niveau d'une des dites gorges, là où la tige présente une section moindre. Facultativement les cannelures ou gorges circulaires pourraient être disposées sur toute la longueur de la broche, participant alors à un effet d'immobilisation de celle-ci dans le milieu osseux.

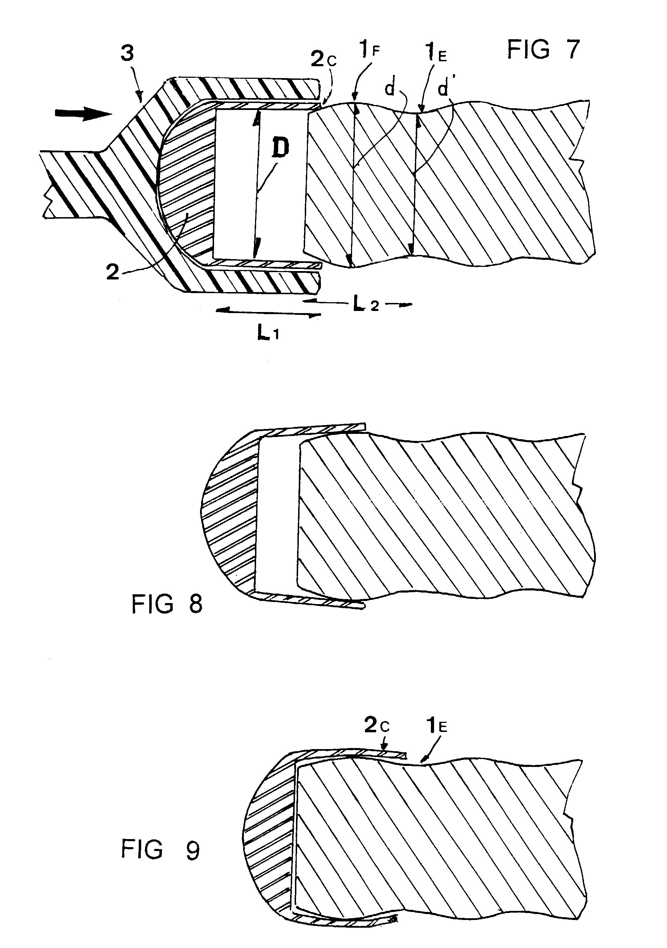

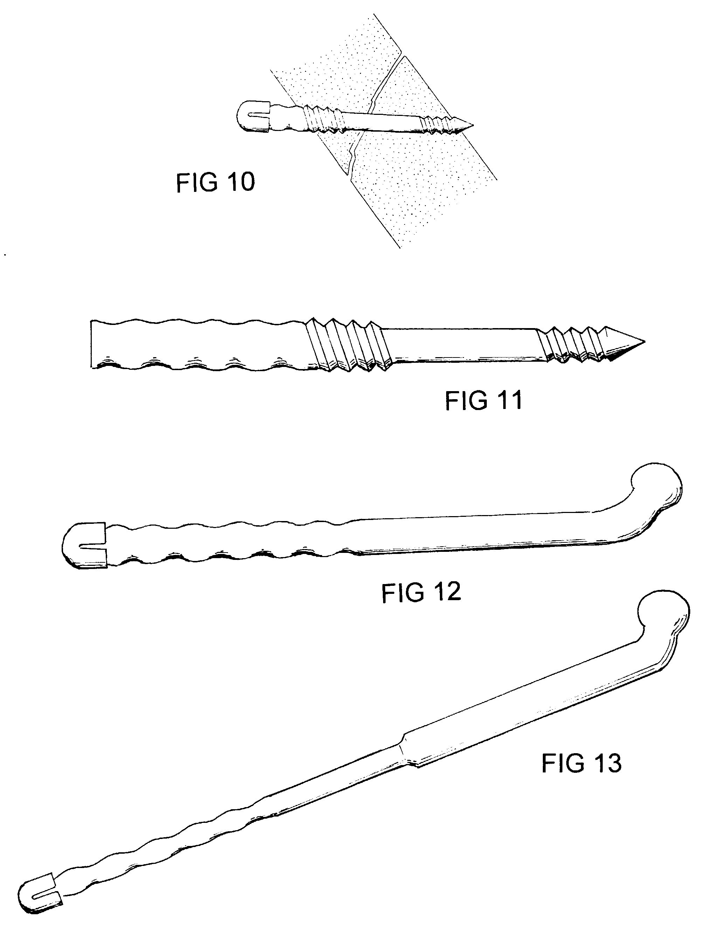

Le capuchon (2) comporte un fond , dont est solidaire une paroi cylindrique (2C) pourvue de fentes ( 2b, 2B), procurant à la dite paroi en forme de jupe une possibilité de déformation diamétrale, propre à faciliter l'engagement du capuchon sur l'extrémité de la tige après sectionnement; les segments de cylindres de la paroi, séparés par les fentes (2b, 2B) étant aptes à s'écarter pour permettre le passage sur cette extrémité, comme représenté à la figure 8. Le diamètre intérieur (D) de l'alésage du capuchon est au plus égal ou de préférence légèrement inférieur au diamètre (d) constituant le diamètre courant de la tige et des collerettes (1e, 1F). Ainsi après passage des segments de cylindres définissant ensemble la paroi du capuchon sur la partie terminale de la tige, par écartement diamétral comme vu à la figure 8, les parois du capuchon se referment au niveau de la première cannelure ou gorge (1E), selon la figure 9, le capuchon étant ainsi serti sur l'extrémité de la tige par ses parois resserrées sur la gorge (1E).

De préférence la mise en place du capuchon s'opère avec l'aide d'une pince ou d'un organe de préhension (3), apte à maintenir les parois du capuchon et à faciliter l'emboîtement en permettant l'expansion diamétrale des parois du capuchon.

Avantageusement le capuchon (2) est réalisé dans le même matériau que la tige (1), par exemple en titane ou acier inoxydable.

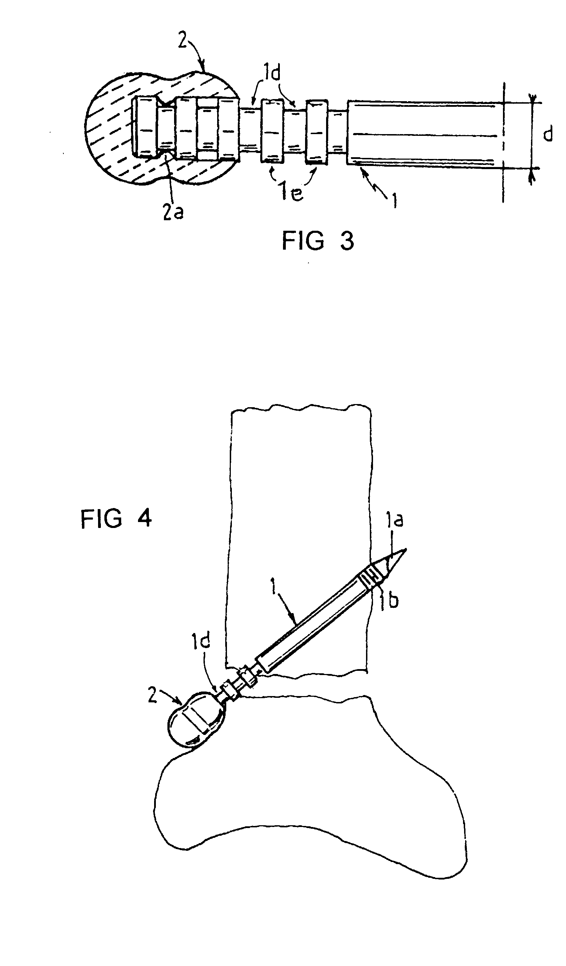

Selon une variante de réalisation de l'invention, telle que représentée à la figure 3, le capuchon comporte dans son alésage une couronne intérieure (2a) , apte à venir s'engager dans une des gorges , de préférence la gorge la plus proche de l'extrémité sectionnée de la tige. Cette couronne a de préférence un profil en V ou curviforme pour faciliter son passage sur la tranche formée par cette extrémité, les parois du capuchon s'écartant élastiquement jusqu'à ce que la couronne interieure (2a) s'insére dans une gorge receptrice, en bloquant ainsi le capuchon en position. Contrairement à ce qui est représenté à la figure 3, la couronne sera de préférence située à proximité de l'embouchure du capuchon, là où la possibilité d'écartement est la plus grande; la couronne pourrait même être constituée par un repli ou rebord vers l'intérieur de l'extrémité du capuchon, venant s'engager dans une gorge.

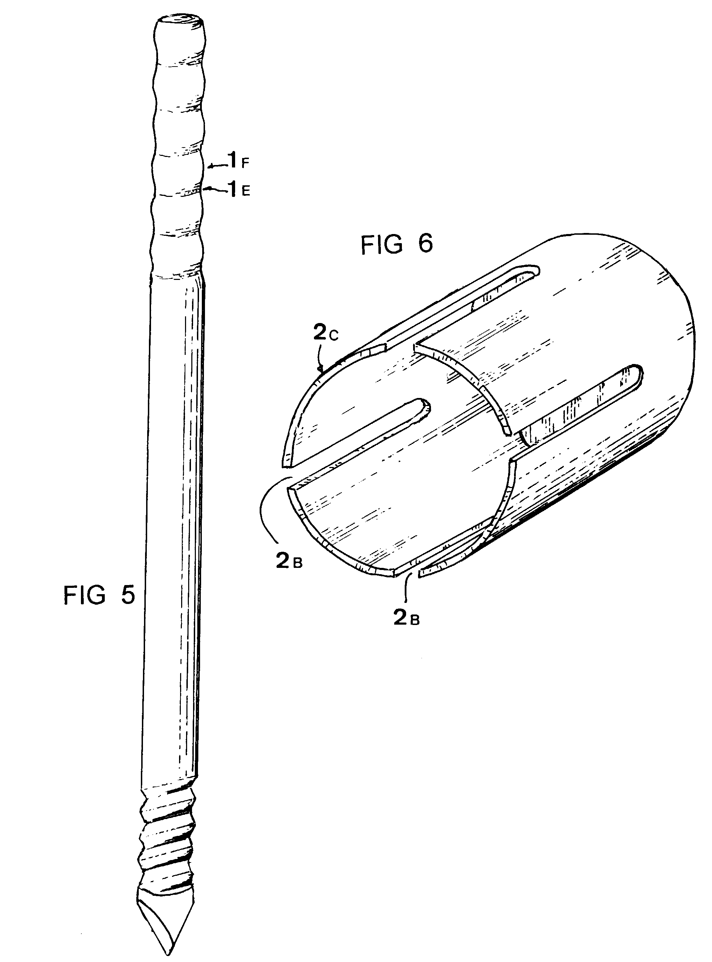

Selon les figures 5 et suivantes, les gorges ou cannelures circulaires et disposées transversalement par rapport à l'axe de la tige sont de profil curviforme concave (1E) ou eventuellement en V, séparées par des collerettes curviformes de profil convexe (1F) de diamètre (d) égal au diamètre courant de la tige sur sa partie lisse. Les profils respectivement concaves des gorges et convexes des collerettes sont identiques et inversés, de sorte que les parois de la tige sur cette partie terminale, vues en coupe, suivent des lignes sinusoïdales symétriques.

Le capuchon de protection (2) constitue par ses parois en forme de jupe, venues d'un fond transversal, un logement borgne propre à recevoir l'extrémité sectionnée de la tige. Le diamètre intérieur (D) de l'alésage de ce logement est au plus égal et de préférence légèrement inférieur au diamètre (d) de chaque collerette (1F), correspondant au diamètre (d) courant de la tige. Ce diamètre intérieur (D) du capuchon est cependant supérieur au diamètre (d') de la tige dans sa zone rétrécie constituant la dite gorge (1E).

Ainsi, comme représenté à la Figure 7, lors de sa mise en place l'embouchure du capuchon s'engage aisément sur extrémité terminale de la tige sectionnée au niveau d'une gorge (lE) dont le diamètre (d') est inférieur au diamètre (D) de la dite embouchure. Les bavures ou déformations métalliques, débordant latéralement, suite à l'écrasement de la matière lors du sectionnement de la tige par pincement, ne gênent pas, puisque la différence des diamètres (d') et (D) laisse un jeu suffisant pour ce passage; cette disposition règle définitivement et de façon nouvelle le problème posé et non résolu dans les dispositifs antérieurs et lié à l'obstacle que constitue la déformation de la tige sur son extrémité sectionnée, lors du passage d'un capuchon de diamètre sensiblement identique à celui de la tige dans le cadre des dispositifs connus.

Lors de la phase d'enfoncement, comme représenté aux Figures 7 et 8, l'embouchure du capuchon s'appuie sur les rampes que constituent les faces ascendantes de la collerette (1F) et par effet de coin, l'enfoncement du capuchon provoque l'écartement des parois du capuchon. Après passage sur la collerette (1F), l'embouchure du capuchon tend à reprendre son diamètre; cette embouchure se resserre et se trouve ainsi sertie autour de la première gorge (1E), assurant l'immobilisation du capuchon coiffant l'extrémité de la tige comme représenté sur la Figure 9. sertie autour de la première gorge (1E), assurant l'immobilisation du capuchon coiffant l'extrémité de la tige comme représenté sur la Figure 9.

L'alésage intérieur du capuchon est prévu avec une profondeur (L1) égale au pas ou distance (L2) séparant le fond de deux gorges successives. Cette disposition permet de s'assurer que la tige ayant tout naturellement et plus facilement été sectionnée au niveau d'un creux d'une gorge, et le fond du capuchon venant donc buter à ce niveau, l'embouchure des parois du capuchon se retrouvera automatiquement au niveau du creux de la première gorge suivant celle où est intervenu le sectionnement; les parois du capuchon pourront ainsi se refermer et procurer la fixation par sertissage décrite ci-dessus. Facultativement la distance (L2) pourrait être un multiple de la distance (L1), l'embouchure du capuchon venant toujours se loger au niveau d'un creux d'une gorge.

L'invention concerne également le procédé de mise en oeuvre de la broche ci-dessus décrite et comportant les étapes suivantes : a) on engage la tige dans l'os ou partie d'os concerné selon la longueur appropriée b) on sectionne l'extrémité terminale de la tige au niveau du creux d'une gorge, c) on insère le capuchon dans une pince ou organe de préhension comportant un logement récepteur du capuchon et propre à maintenir le capuchon, d) on engage l'embouchure du capuchon sur l'extrémité sectionnée de la tige, e) on repousse le capuchon en l'enfonçant sur la tige, les parois du capuchon s'écartant diamétralement en remontant sur la rampe conique que constitue la face ascendante de la collerette jusqu'à ce que le fond du capuchon vienne en butée sur l'extrémité sectionnée de la tige, f) on retire l'organe de préhension, la fixation du capuchon étant obtenue par une pression centripète exercée sur l'extrémité de la tige par les parois du capuchon.

Le diamètre extérieur du capuchon étant, par définition, supérieur au diamètre de la tige, le capuchon fait ainsi office de butée évitant tout risque de migration vers l'intérieur de l'os . Le capuchon a, de façon connue en soi, des formes extérieures arrondies et non agressives.

La fixation du capuchon étant obtenue par emboîtement sous pression et sertissage de ses parois sur l'extrémité de la tige, le retrait du capuchon, si nécessaire, reste cependant possible par le mouvement inverse; une traction exercée sur le bouchon vers l'arrière provoquera l'ouverture de son embouchure par l'effet de rampe exercée par la face en regard de la collerette, ce qui permet de retirer éventuellement le dit capuchon; la mise en place du capuchon est ainsi réversible.

Outre sa fonction de protection et de butée, le capuchon permet de localiser plus facilement la broche lorsqu'elle doit être retirée; la présence des gorges ou cannelures assurera par ailleurs une meilleure prise pour une pince lors de l'extraction de la tige.

Les avantages du dispositif ressortent ainsi clairement de la description qui précède; essentiellement la combinaison des gorges ou cannelures et du capuchon à parois diamétralement expansible permet de supprimer la difficulté née de la présence sur l'extrémité sectionnée de la tige de bavures ou déformation gênant le passage et la mise en place d'un capuchon.

Cette mise en place est obtenue par un mouvement unidirectionnel de simple pression, pratiquement instantané, assurant par sertissage ou effet de clip l'immobilisation réversible du capuchon sur l'extrémité de la tige, en évitant tout risque de retrait . Cette simplicité de manoeuvre est particulièrement utile dans une zone peu accessible chirurgicalement.

Les fonctions connues du capuchon restent ainsi parfaitement assurées, notamment la protection de l'extrémité de la tige évitant toute agression sur les tissus, l'effet de butée au niveau de la corticale de l'os, supprimant pendant la période de reconstitution ou d'ostéosynthèse, qu'à une préhension plus aisée de la tige à l'occasion de son extraction.

La déformation diamétrale des parois cylindriques du capuchon peut être obtenue par l'élasticité de la matière elle même, les parois du capuchon s'ouvrant élastiquement pour passer sur la collerette (1F) et se refermant ensuite elastiquement autour de la gorge suivante (1E).

Avantageusement l'organe de préhension (3) pour la mise en place du capuchon définit un logement, dans le fond duquel vient en butée d'appui la face externe arrière du capuchon, et les bords du dit logement sont constitués de doigts élastiquement deformables (3A), ou eventuellement articulés, et constituant des mors, propres à maintenir les parois du capuchon, ces doigts s'écartant (éventuellement par élasticité) lors du passage des parois (2C) du capuchon sur la collerette (1F, Figure 7 et 8). Après quoi lesdites parois se resserrent autour de la gorge (1E, Figure 9) procurant la fixation par sertissage précédemment décrit. Un sertissage final peut encore être obtenu par la manoeuvre d'une pince indépendante de l'organe de préhension.

L'invention s'applique à tout type de broche, quelles qu'en soient les formes, dimensions ou applications, ceci dans le cadre de la chirurgie traumatologique et orthopédique pour toute partie de corps dans les familles des vertébrés.

Ainsi les figures 9 et 10 représentent l'application du dispositif de l'invention à une broche à double filetage, le diamètre du filetage arrière étant supérieur à celui situé sur l'extrémité avant, ainsi propre à assurer un effet de compression sur un foyer osseux mobile.

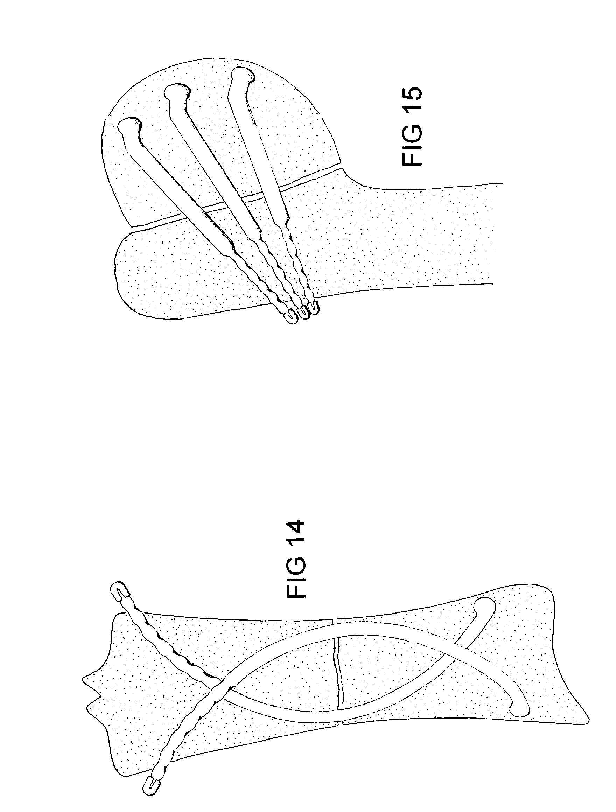

Les figures 12 et 14 représentent des broches pourvues à une extrémité du dispositif de protection de l'invention et présentant à leur extrémité opposée une conformation boutonnée et béquillée, permettant de traiter avec un maximum de sécurité les fractures des os longs de l'enfant et de l'adulte.

Et les figures 13 et 15 représentent des broches dont une extrémité renflée se termine par un bouton béquillé, l'autre extrémité, rétrécie, reçoit le dispositif de protection de l'invention, cette conformation permettant de traiter plus efficacement les fractures métaphysaires des os longs.

## Revendications

1. Broche d'ostéosynthèse du type constitué d'une part d'une tige cylindrique (1) apte à être introduite dans l'os ou partie d'os concerné à partir de son extrémité de perforation profilée à cet effet, et d'autre part d'un capuchon de protection (2) apte à être engagé sur l'extrémité terminale de la dite tige opposée à son extrémité d'introduction, après mise en place de la tige, caractérisée a) en ce que la dite tige comporte au moins sur la dite extrémité terminale, opposée à l'extrémité de perforation et destinée à recevoir le capuchon, des inégalités de relief sous forme de cannelures ou gorges circulaires (1d, 1E), transversales par rapport à l'axe de la tige et b) en ce que le capuchon, constitué d'un fond et de parois (2C) en forme de jupe, apte à s'emboîter sur la dite extrémité, est prévu avec des parois déformables diamétralement, ainsi propres à s'ouvrir pour s'engager sur la dite extrémité et à s'y fixer en s'emboîtant sous pression sur ladite extrémité.

2. Broche d'ostéosynthèse selon la revendication 1 et caractérisée en ce que les gorges circulaires (1d, lE) sont reparties de façon équidistante au moins sur la dite partie terminale de la tige cylindrique.

3. Broche d'ostéosynthèse selon la revendication 2 et caractérisée en ce que les gorges circulaires (1E) sont de profil concave curviforme ou en V.

4. Broche d'ostéosynthèse selon la revendication 2 et la revendication 3 et caractérisée en ce que les gorges sont séparées par des collerettes (1F), de diamètre (d) égal au diamètre courant de la tige cylindrique, les profils respectivement concaves des gorges et convexes des collerettes étant identiques et inversés.

5. Broche d'ostéosynthèse selon la revendication 4, caractérisée en ce que le capuchon de protection (2) définit un logement cylindrique borgne propre à recevoir l'extrémité de la tige après sectionnement à bonne longueur de cette dernière et le diamètre (D) intérieur du dit logement est compris entre le diamètre minimal (d') des dites gorges (1E) et le diamètre (d) maximal de la collerette (1F), permettant l'engagement aisé de l'embouchure du capuchon sur l'extrémité de la tige, préalablement sectionnée au niveau de ce diamètre minimal de la gorge, l'engagement ultérieur du capuchon provoquant la déformation et l'ouverture de ses parois portant sur la rampe ascendante formée par la face en regard de la dite collerette (1F).

6. Broche d'ostéosynthèse selon l'une des revendications 1 à 5 et caractérisé en ce que le dit capuchon présente sur ses parois des fentes latérales (2b, 2B) permettant la déformation et l'écartement diamétral des parois lors de l'engagement du capuchon sur l'extrémité de la tige et notamment sur la dite rampe ascendante constituée par la collerette (1F) suivi du resserrement ultérieur des parois après passage sur la collerette.

7. Broche d'ostéosynthèse selon l'une des revendications 1 à 6, caractérisée en outre en ce que l'alésage intérieur du capuchon est prévu avec une profondeur (L1), comprise entre son embouchure et son fond, égale à la distance (L2) séparant deux gorges, ou un multiple de cette distance, de sorte que la tige ayant été sectionnée au creux d'une gorge et le fond du capuchon venant en butée sur cette extrémité sectionnée, l'embouchure du capuchon se situe alors au niveau de la gorge faisant suite à la gorge sectionnée, permettant à cette embouchure de se refermer par sertissage autour du creux de cette gorge.

8. Broche d'ostéosynthèse selon l'une des revendications 1 à 7, caractérisée en ce qu'elle est associée à un organe de préhension (3), telle qu'une pince de mise en place et de sertissage et comportant un logement récepteur du capuchon, l'embouchure de ce dernier restant dégagée, et les bords de cet organe de préhension forment des mors (3A) venant porter sur les parois externes (2C) du capuchon et ils sont prévus aptes à maintenir ces parois (2C) tout en permettant leur expansion diamétrale lors du passage du capuchon autour d'une collerette de l'extrémité terminale de la tige.

9. Broche d'ostéosynthèse selon l'une des revendications 1, 2 ou 3 et caractérisée en ce que le capuchon (2) comporte sur son alésage intérieur une couronne interne (2a), apte à s'engager dans une gorge (1d), assurant ainsi la fixation du capuchon .

10. Broche d'ostéosynthèse selon l'une des revendications 1 à 10, caractérisée en ce que le capuchon est réalisé en un matériau permettant une déformation élastique.

11. Procédé de mise en oeuvre d'une broche d'ostéosynthèse selon l'une des revendications 1 à 10 qui précédent et caractérisé par la succession des étapes suivantes : a) on engage la tige dans l'os ou partie d'os concerné selon la longueur appropriée, b) on sectionne l'extrémité terminale de la tige au niveau d'un creux d'une gorge, c) on engage l'embouchure du capuchon, monté sur une pince appropriée, sur cette extrémité sectionnée, d) on repousse le bouchon sur la tige selon l'axe de cette dernière, les parois du capuchon s'ouvrant diamétralement par effet de coin, en portant sur la face ascendante de la collerette voisine, e) on assure la fixation du capuchon sur cette extrémité par pression centripète des parois du capuchon sur l'extrémité de la tige, le fond du capuchon étant alors en butée sur cette extrémité, f) on retire la pince, le capuchon restant en place par pression de ses parois sur l'extrémité de la tige.
